# EUROPÄISCHE PATENTSCHRIFT

(11) **EP 1 617 784 B1**
(45) Veröffentlichungstag und Bekanntmachung des Hinweises auf die Patenterteilung: **20.09.2006**
(21) Anmeldenummer: 04728348.6
(22) Anmeldetag: 20.04.2004
(51) Int. Cl.: A61F 2/00

(54) **CHIRURGISCHE FLÄCHENEINLAGE**
SURGICAL PLANAR PLUG
PIECE D'INSERTION CHIRURGICALE PLANE

(30) Priorität: 28.04.2003 DE 20306635 U
(43) Veröffentlichungstag der Anmeldung: 25.01.2006
(73) Patentinhaber: GfE Medizintechnik GmbH, 90431 Nürnberg (DE)
(72) Erfinder: ZIMMERMANN, Hanngörg, 90574 Rosstal (DE); FRICKE, Helmut, 38536 Meinersen-Ahnsen (DE)
(74) Vertreter: Hübner, Gerd
(86) Internationale Anmeldenummer: PCT/EP2004/004169
(87) Internationale Veröffentlichungsnummer: WO 2004/096086

(56) Entgegenhaltungen:
- EP-A- 0 897 997
- EP-A- 1 087 034
- WO-A-92/10218
- WO-A-93/17635
- DE-A- 10 221 320
- DE-C- 4 013 447
- US-B1- 6 319 264
- ANONYMUS: "Surgi-Wrap Bioresorbierbare Anti-Adhesionsfolie" [Online] 2002, MACRO PORE BIOSURGERY INC , SAN DIEGO,US , XP002297528 Gefunden im Internet: URL:http://www.fumedica.com/macro_pore/mac ropore.html> in der Anmeldung erwähnt das ganze Dokument

## Beschreibung

Die Erfindung betrifft eine chirurgische Flächeneinlage zur Verhinderung von Gewebelagenverwachsungen in Operationsbereichen, insbesondere zur postoperativen Versorgung bei perikardialen, peritonialen oder gynäkologischen Eingriffen, nach dem Oberbegriff des Anspruches 1, wie aus WO 9210218 bekannt.

Derartige Flächeneinlagen sind in der Chirurgie bekannt und weisen eine Lage einer dünnen, bioresorbierbaren, glatten Folie auf, die als Adhäsionsbarriere gegen die erwähnten Gewebelagenverwachsungen fungieren.

Letztere bestehen aus fibrösen Bändern, die abnormale Verbindungen zwischen zwei Körper-Gewebelagen bilden, die normalerweise getrennt sind. Derartige Adhäsionen oder sogenanntes Narbengewebe kann zu einer Vielzahl von Komplikationen führen, wie schweren Unterleibsschmerzen, Unfruchtbarkeit oder Darmverschlüssen. Die Verwachsungen beruhen auf einer Zellreaktion nach einer traumatischen Verletzung von Gewebe, wie es bei chirurgischen Eingriffen stattfindet. Nach den Trauma beginnt sich im Gewebe eine Fibrinmatrix zu bilden. Fibroplasten, Fibrinfäden und Histamine wuchern. Mit der Zeit bilden sich Fibrinbänder zwischen zwei Gewebelagen, wobei die resultierenden Adhäsionskräfte das umgebende Gewebe zusammenziehen, was gegenteilige Effekte bewirkt. Insgesamt ist der lagige Körperaufbau in Operationszonen erheblich gestört.

Zur Vermeidung der vorstehenden Probleme werden bioresorbierbare Barrierefolien in die Operationsbereiche eingelegt, womit die Adhäsionsbildung unterbunden und ferner chirurgische Trennebenen für etwaige Nachoperationen gebildet werden.

Die Barrierefolien sind ultradünn und sehr leicht verformbar, so dass sie sich der Körperumgebung gut anpassen. Dieser Effekt ist in aller Regel erwünscht. Es treten jedoch Anwendungsfälle auf, bei denen die Notwendigkeit einer höheren Stabilität besteht, in denen der Operationsbereich beispielsweise zur Narbenstützung zusätzlich stabilisiert werden soll. Hier bietet die Folie zu wenig Unterstützung.

Davon ausgehend liegt der Erfindung die Aufgabe zugrunde, eine chirurgische Flächeneinlage zur Verhinderung von Gewebelagenverwachsungen in Operationsbereichen zu schaffen, die eine erhöhte mechanische Stabilität aufweist.

Diese Aufgabe wird durch die im Kennzeichnungsteil des Anspruches 1 angegebenen Merkmale gelöst. Demnach ist die bioresorbierbare Folienlage mit einer Stabilisierungslage in Form eines Verstärkungsnetzes aus Kunststoffmaterial verbunden, das mit einer metallhaltigen, körperverträglichen, durchgehenden Beschichtung versehen ist. Derartige Verstärkungsnetze sind als separate Produkte auf dem Gebiet der Medizintechnik an sich bekannt, nämlich als sogenannte Herniennetze, wie sie zur Stabilisierung der Bauchdecke bei Leistenbruchoperationen eingesetzt werden.

Die erfindungsgemäße Flächeneinlage weist also eine Verbundstruktur auf, wobei die beiden Lagen jeweils spezifische Funktionen erfüllen. Die bioresorbierbare Folie verhindert die Gewebelagenverwachsungen während einer postoperativen Phase und wird mit der Zeit abgebaut. Das Verstärkungsnetz stabilisiert von Anfang an den Operationsbereich, verbleibt nach dem Abbau der Barrierefolie jedoch im Körper, um die Stabilisierungsfunktion dauerhaft fortzusetzen.

Im Zusammenhang mit dem Verbleib des Verstärkungsnetzes im Körper ist die metallhaltige, körperverträgliche, durchgehende Beschichtung von besonderer Bedeutung.

Diese Beschichtung, die vorzugsweise auf der Basis Titan-haltiger Verbindungen mit einer Dicke von < 2 µm, vorzugsweise von 5 bis 700 nm hergestellt ist, erfüllt eine Vielzahl von Funktionen. So erfolgt durch ihre Durchgängigkeit kein direkter Kontakt des Basis-Kunststoffmaterials des Verstärkungsnetzes mit Körpergewebe mehr, so dass das Netz vom Körper durchgängig als aus einem vorzugsweise Titan-haltigen Material bestehend wahrgenommen wird. Dadurch werden Abstoßungsreaktionen in Form einer Verkapselung des Netzes nicht in Gang gesetzt. Ferner stellt nachweisbar die metallhaltige Beschichtung eine Diffussionssperre für die Weichmachermoleküle im Kunststoffmaterial des Netzes dar, so dass ein Herausdiffundieren der Weichmacher unterbunden wird. Damit wird eine Versprödung des Netzes minimiert.

Die metallhaltige Beschichtung besteht bevorzugt aus einer Verbindung der Formel TiₐO_{b}C_{c}, wobei gilt
a = 0,025 bis 0,9,
b = 0,025 bis 0,7 und
c = 0,2 bis 0,9.

Ggf. können im Austausch zu den Titan-Anteilen auch Tantal, Niob, Silber, Zirkon und Hafnium eingesetzt werden. Als weitere Elemente in der Verbindung können ferner Stickstoff und Bor beinhaltet sein.

Das Kunststoffmaterial des Verstärkungsnetzes besteht vorzugsweise aus Polypropylen, Polyurethan, Polypolyester oder PTFE. Die bioresorbierbare Folie ist auf der Basis von Polylactat hergestellt.

Die Verbindung der Folienlage mit der Stabilisierungslage in Form des Verstärkungsnetzes kann beispielsweise mittels einer punktweisen Verklebung etwa an den Kreuzungspunkten des Netzes mit der Folie erfolgen. Bevorzugt wird dann ein bioresorbierbarer oder zumindest körperverträglicher Klebstoff, beispielsweise Fibrin-Kleber, verwendet. Eine Alternative dazu kann die mechanische Verbindung mit Hilfe von Verknotungsfäden sein, die dann wie das Verstärkungsnetz selbst mit der durchgehenden körperverträglichen, metallhaltigen Beschichtung versehen sind.

Schließlich kann in das Verbundsystem als dritte Komponente noch eine Hämostyptikum-Lage zur Abgabe blutstillender Mittel eingebaut sein.

Weitere Merkmale, Einzelheiten und Vorteile der Erfindung ergeben sich aus der nachfolgenden Beschreibung, in der Ausführungsbeispiele anhand der beigefügten Zeichnungen näher erläutert werden. Es zeigen:
- Fig. 1: eine schematische Perspektivdarstellung einer chirurgischen Flächeneinlage, sowie
- Fig. 2 und 3: vergrößerte Detailschnitte quer zur Lagenebene zweier unterschiedlicher Ausführungsformen der Flächeneinlage.

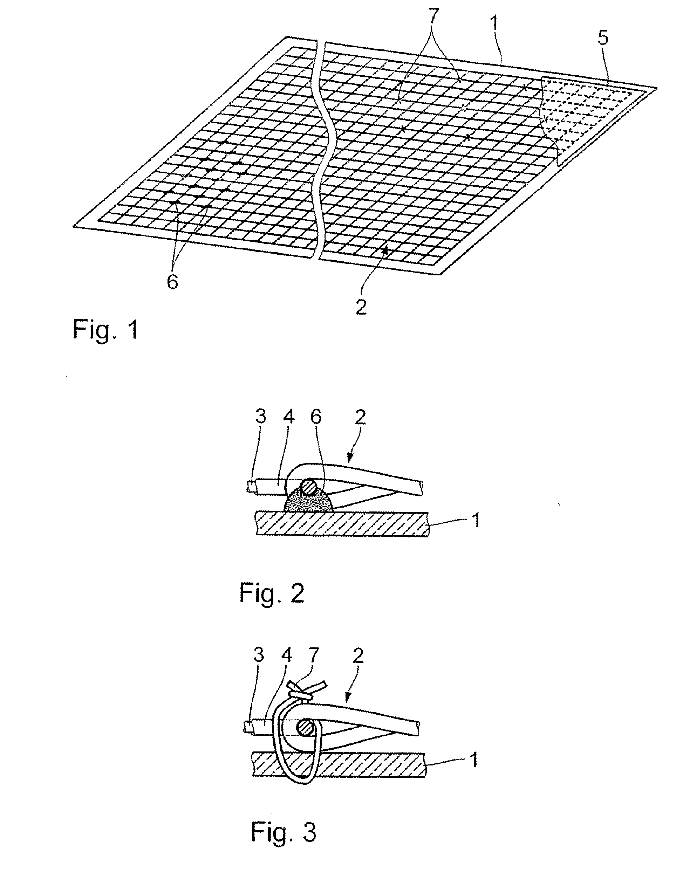

Wie aus Fig. 1 deutlich wird, besteht die Flächeneinlage aus einer dünnen, bioresorbierbaren, glatten Folienlage 1, die auf der Basis eines Polylactats hergestellt ist. Deren Dicke beträgt beispielsweise 0,02 oder 0,05 mm. Die Flächendimensionierung liegt im Bereich zwischen 70 und 200 mm in der Länge sowie 50 und 130 mm in der Breite. Derartige Folien werden beispielsweise unter der Markenbezeichnung "Surgi wrap" ® von der Firma MacroPore Biosurgery, Inc, San Diego, CA, USA vertrieben. Nach dem Einsetzen der Flächeneinlage in den menschlichen Körper löst sich die Folienlage 1 aufgrund ihrer bioresorbierbaren Eigenschaften typischer Weise innerhalb etwa drei Monate auf.

Auf der Folienlage 1 ist ein Verstärkungsnetz 2 aufgebracht, das aus einem Kunststofffaden 3 beispielsweise aus Polypropylen, Polyurethan, Polyester oder PTFE gewirkt ist. Wie aus Fig. 2 und 3 deutlich wird, ist dieser Kunststofffaden 3 auf seiner Außenseite mit einer durchgehenden, körperverträglichen Beschichtung 4 aus einem Titan-haltigen Material der Formel TiₐO_{b}C_{c} versehen.

Die Anteilsbereiche a, b und c entsprechen denen in der Beschreibungseinleitung. Eine derartige Beschichtung hat sich als absolut körperverträglich herausgestellt. Durch ihre Durchgängigkeit wird die gesamte Seele des Fadens 3 mit ihrem Kunststoffmaterial nicht mehr als solches vom Körper wahrgenommen. Die Körperverträglichkeit ist somit mit komplett aus einer Titanlegierung gefertigten Implantaten vergleichbar, die in der Medizintechnik breite Akzeptanz finden.

Bevorzugte Dicken der Beschichtung 4 liegen im Bereich von 5 bis 700 nm, wobei Schichtdicken von etwa 50 nm sich als einerseits besonders haft- und reibfest, andererseits aber auch genügend flexibel und duktil herausgestellt haben, um alle Dehnungen und Verformungen des Fadens und damit des Verstärkungsnetzes schadlos mit zu vollführen.

Derartige Titan-haltige Beschichtungen und die Technik zum Aufbringen auf flexible Kunststoffsubstrate sind im Übrigen aus dem Stand der Technik, wie etwa der EP 0 897 997 B1, grundsätzlich bekannt.

Ein Verstärkungsnetz 2 als solches und dessen Realisierung als Gewirk aus einem metallbeschichteten Kunststofffaden 3 sind im Übrigen eingehend in der älteren deutschen Patentanmeldung 102 21 320.8 (veröffentlicht als DE 102 21 320 A) der Anmelderin beschrieben.

Wie in Fig. 1 ferner angedeutet ist, kann als weitere Komponente des flächigen Verbundes eine Hämostyptikum-Lage 5 auf der Außenseite der Flächeneinlage, also auf dem Verstärkungsnetz 2, aufgebracht sein (siehe Fig. 1, Ecke rechts außen). Diese Hämostyptikum-Lage beinhaltet ein blutstillendes Mittel, das mit der Zeit beispielsweise ebenfalls durch Resorption der Lage 5 an den Körper abgegeben werden kann.

Zu der Lagenstruktur ist im Übrigen festzuhalten, dass Verstärkungsnetz und Folienlage mehrfach und in unterschiedlichen Schichtungen vorgesehen sein können. So kann ein zentrales Verstärkungsnetz mit zwei beidseitigen Folienlagen 1 oder umgekehrt eine Folienlage 1 mit zwei beidseitigen Verstärkungsnetzen 2 kombiniert werden. Auch Sandwich-Strukturen mit mehreren Folienlagen 1 und Verstärkungsnetzen 2 in abwechselnder Schichtung sind je nach Einsatzweck realisierbar.

Zur Verbindung der einzelnen Lagen können verschiedene Techniken angewandt werden, die in Fig. 1 schematisch angedeutet sind. Im linken Teil der Fig. 1 und in Fig. 2 sind punktweise Verklebungen 6 erkennbar, die an den Kreuzungspunkten des Verstärkungsnetzes sitzen. Bei den Verklebungen 6 kann es sich beispielsweise um Tropfen eines Fibrin-Klebstoffes handeln, der biokompatibel ist.

Eine alternative Verbindungsweise ist in Fig. 1, rechter Teil und Fig. 3 dargestellt, nämlich die punktweise Verbindung des Verstärkungsnetzes 2 mit der Folienlage 1 mittels Verknotungsfäden 7, die jeweils in Kreuzungspunkten des Verstärkungsnetzes 2 und durch die Folienlage 1 nach Art eines Heftstiches durchgeführt und verknotet werden. Die Verknotungsfäden 7 selbst sind wieder mit der bereits beschriebenen, körperverträglichen, metallhaltigen Beschichtung auf Titan-Basis versehen, so dass auch diese keine Körperabwehrreaktionen hervorrufen.

## Patentansprüche

1. Chirurgische Flächeneinlage zur Verhinderung von Gewebelagenverwachsungen in Operationsbereichen, insbesondere zur postoperativen Versorgung bei pericardialen, peritonealen oder gynäkologischen Eingriffen, umfassend
- mindestens eine Lage (1) einer dünnen, bioresorbierbaren, glatten Folie **gekennzeichnet durch**
- eine mit der Folienlage (1) verbundene Stabilisierungslage in Form eines Verstärkungsnetzes (2) aus Kunststoffmaterial, das mit einer metallhaltigen, körperverträglichen, durchgehenden Beschichtung (4) versehen ist.

2. Chirurgische Flächeneinlage nach Anspruch 1, **dadurch gekennzeichnet, dass** die Beschichtung (4) eine Titan-haltige Beschichtung mit einer Dicke von kleiner 2 µm, vorzugsweise von 5 bis 700 nm ist.

3. Chirurgische Flächeneinlage nach Anspruch 2, **dadurch gekennzeichnet, dass** die Beschichtung (4) eine Verbindung der Formel
TiₐO_{b}C_{c}
aufweist, wobei gilt a = 0,025 bis 0,9
b = 0,025 bis 0,7 und
c = 0,2 bis 0,9.

4. Chirurgische Flächeneinlage nach einem der vorgenannten Ansprüche, **dadurch gekennzeichnet, dass** das Verstärkungsnetz (2) aus Polypropylen, Polyurethan, Polyester oder PTFE besteht.

5. Chirurgische Flächeneinlage nach einem der vorgenannten Ansprüche, **dadurch gekennzeichnet, dass** die bioresorbierbare Folienlage (1) aus einem Polylactat besteht.

6. Chirurgische Flächeneinlage nach einem der vorgenannten Ansprüche, **dadurch gekennzeichnet, dass** das Verstärkungsnetz (2) mittels punktweiser Verklebungen (6) mit der Folienlage (1) verbunden ist.

7. Chirurgische Flächeneinlage nach einem der Ansprüche 1 bis 5, **dadurch gekennzeichnet, dass** das Verstärkungsnetz (2) punktweise mit der Folienlage (1) mittels Verknotungsfäden (7) verbunden ist, die ebenfalls mit der durchgehenden körperverträglichen, metallhaltigen Beschichtung versehen sind.

8. Chirurgische Flächeneinlage nach einem der vorgenannten Ansprüche, **dadurch gekennzeichnet, dass** eine Hämosteptikum-Lage (5) zur Abgabe blutstillender Mittel vorzugsweise auf der Außenseite der Flächeneinlage (1) vorgesehen ist.

## Claims

1. A surgical flat implant for preventing tissue-to-tissue adhesion in operated areas, in particular for post-operative repair in pericardial, peritoneal or gynaecological surgery, comprising
- at least one layer (1) of a thin, bioresorbable, smooth film,
**characterized by**
- a stabilizing layer in form of a reinforcing mesh (2) of plastic material which is joined to the film layer (1) and which is provided with a metal-containing, biocompatible, continuous coating (4).

2. A surgical flat implant according to claim 1, **characterized in that** the coating (4) is a titanium-containing coating of a thickness of less than 2 µm, preferably of 5 to 700 nm.

3. A surgical flat implant according to claim 2, **characterized in that** the coating (4) comprises a compound of the formula
TiₐO_{b}C_{c},
with a = 0.025 to 0.9,
b = 0.025 to 0.7 and
c = 0.2 to 0.9
applying.

4. A surgical flat implant according to one of the preceding claims, **characterized in that** the reinforcing mesh (2) consists of polypropylene, polyurethane, polyester or PTFE.

5. A surgical flat implant according to one of the preceding claims, **characterized in that** the bioresorbable film layer (1) consists of a polylactate.

6. A surgical flat implant according to one of the preceding claims, **characterized in that** the reinforcing mesh (2) is joined to the film layer (1) by glued spots (6).

7. A surgical flat implant according to one of claims 1 to 5, **characterized in that** the reinforcing mesh (2) is joined to the film layer (1) by spots by means of knotted filaments (7) which are also provided with the continuous, biocompatible, metal-containing coating.

8. A surgical flat implant according to one of the preceding claims, **characterized in that** a hemostyptic layer (5) for hematostatic-agent release is provided preferably on the outside of the flat implant (1).

## Revendications

1. Pièce d'insertion chirurgicale plane pour éviter des adhérences de tissus dans des zones opératoires, notamment pour les soins postopératoires après des interventions péricardiques, péritonéales ou gynécologiques, comprenant
- au moins une couche (I) d'un film mince biorésorbable,
**caractérisée par**
- une couche de stabilisation reliée avec la couche de film (1) sous la forme d'un filet de renfort (2) en matière plastique, qui est muni d'un revêtement continu (4), biocompatible, contenant du métal.

2. Pièce d'insertion chirurgicale plane selon la revendication 1, **caractérisée en ce que** le revêtement (4) est un revêtement contenant du titane, d'une épaisseur inférieure à 2 µm, de préférence de 5 à 700 nm.

3. Pièce d'insertion chirurgicale plane selon la revendication 2, **caractérisée en ce que** le revêtement (4) présente un composé de la formule
TiₐO_{b}C_{c}
avec a = 0,025 à 0,9
b = 0,025 à 0,7 et
c = 0,2 à 0,9

4. Pièce d'insertion chirurgicale plane selon l'une quelconque des revendications précédentes, **caractérisée en ce que** le filet de renfort (2) est en polypropylène, en polyuréthane, en polyester ou en PTFE.

5. Pièce d'insertion chirurgicale plane selon l'une quelconque des revendications précédentes, **caractérisée en ce que** la couche de film biorésorbable (1) est en un polylactate.

6. Pièce d'insertion chirurgicale plane selon l'une quelconque des revendications précédentes, **caractérisée en ce que** le filet de renfort (2) est relié à la couche de film (1) au moyen de points de collage (6).

7. Pièce d'insertion chirurgicale plane selon l'une quelconque des revendications 1 à 5, **caractérisée en ce que** le filet de renfort (2) est relié par points avec la couche de film (1) par des fils à nouer (7) qui sont également munis du revêtement continu biocompatible contenant du métal.

8. Pièce d'insertion chirurgicale plane selon l'une quelconque des revendications précédentes, **caractérisée en ce qu'**on a prévu une couche hémostyptique (5) pour distribuer des produits styptiques, de préférence sur la face extérieure de la pièce d'insertion plane (1).
